(19) Europäisches Patentamt
European Patent Office
Office européen des brevets

(11) **EP 3 921 221 B1**

(12) **EUROPÄISCHE PATENTSCHRIFT**

(45) Veröffentlichungstag und Bekanntmachung des Hinweises auf die Patenterteilung:
**09.04.2025   Patentblatt 2025/15**

(21) Anmeldenummer: **20713156.6**

(22) Anmeldetag: **18.01.2020**

(51) Internationale Patentklassifikation (IPC):
**B62J 1/00** *(2006.01)*     **A61B 8/08** *(2006.01)*

(52) Gemeinsame Patentklassifikation (CPC):
**B62J 1/007; A61B 8/085; A61B 8/4209; A61B 8/4455**

(86) Internationale Anmeldenummer:
**PCT/DE2020/000005**

(87) Internationale Veröffentlichungsnummer:
**WO 2020/160720 (13.08.2020 Gazette 2020/33)**

(54) **VERFAHREN ZUR HERSTELLUNG EINER INDIVIDUELLEN SATTELSCHALE, SATTELSCHALE, SATTEL UND FAHRRAD MIT SATTEL**

METHOD FOR PRODUCING A CUSTOM SADDLE SHELL, SADDLE SHELL, SADDLE AND BICYCLE WITH SADDLE

PROCÉDÉ DE FABRICATION D'UNE COQUE DE SELLE INDIVIDUELLE, COQUE DE SELLE, SELLE ET BICYCLETTE COMPRENANT UNE SELLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:  **07.02.2019   DE 102019000882**

(43) Veröffentlichungstag der Anmeldung:
**15.12.2021   Patentblatt 2021/50**

(73) Patentinhaber: **Hohmuth, Horst**
**89073 Ulm (DE)**

(72) Erfinder: **Hohmuth, Horst**
**89073 Ulm (DE)**

(74) Vertreter: **Kimpfbeck, Thomas**
**Haydstraße 2**
**85354 Freising (DE)**

(56) Entgegenhaltungen:
WO-A1-2007/147524     DE-U1- 20 000 745
DE-U1- 202015 003 752     US-A- 1 975 405
US-A1- 2004 024 315     US-A1- 2006 218 809
US-A1- 2007 200 399     US-A1- 2010 045 084
US-A1- 2018 017 368     US-B1- 8 187 260

**Beschreibung**

TECHNISCHES GEBIET

Die vorliegende Erfindung betrifft ein Verfahren zur Herstellung einer individuellen Sattelschale.

STAND DER TECHNIK

Es ist bekannt, dass bei männlichen Radsportlern vermehrt Erektionsstörungen auftreten, die durch Fahrradsättel und den damit verbundenen Druck auf den Damm und Penisgefäße verursacht werden. Gerade Männer mit Prostatavergrößerung, die gerne Fahrrad fahren, klagen nach längeren Fahrradtouren über Taubheitsgefühl und Minderdurchblutung im Penis. Dieses Problem wird auch nicht durch die weitverbreiteten Gelsättel beseitigt, denn das Gel wird durch das Körpergewicht verdrängt. Der Mann drückt dann mit seinem Körpergewicht auf eine harten Kunststoffschale des Sattels und damit wird seine Prostata und die Peniswurzel komprimiert.

Zur Entlastung des Genitalbereichs ist aus der EP 3 022 110 A1 etwa ein Sattel mit einem schmalen Kanal bekannt. Dieser Kanal ist trapezförmig in einer Längsachse des Sattels ausgebildet, weist am zu einer hinteren Sattelkante weisenden Ende eine konstante Breite von 30 bzw. 35 mm auf, verläuft über etwa drei Viertel der Sattellänge und verjüngt sich dabei zur Sattelnase hin auf eine konstante Breite von 10 bzw. 20 mm.

Schmale, lange Kanäle in einem Sattel führen im Allgemeinen dazu, dass das Gewicht des Mannes mit einer relativ geringen Auflagefläche auf den Schambeinschaufeln lastet. Dies führt zu einer hohen Druckbelastung der Schambeinschaufeln, was zu chronischen Schmerzen und Knochenhautentzündungen (Periostitis) der Sitz- und Schambeinäste (Tuber ischiaticum und Os pubis) führen kann, welche zudem durch solche Sattelkonstruktionen auch zu Tendinopathien des Musculus piriformis Ansatzes ("ham-string muscle") führen kann. Diese Krankheitsbilder sind in der Sportmedizin gut bekannt und verursachen einen langwierigen Heilungsverlauf. Dieses Krankheitsbild kann unter anderem auch durch Sattelkonstruktionen hervorgerufen werden, bei denen es zu einer punktuell hohen Druckbelastung der Knochenhaut kommt. Das Dokument US 2006/218809 A1 zeigt ein Verfahren zur Herstellung einer Sattelschale.

Somit besteht die Aufgabe der vorliegenden Erfindung darin einen hinsichtlich eines Fahrers optimierten Sattel, ein Verfahren zur Herstellung einer individuellen Sattelschale zu schaffen.

OFFENBARUNG DER ERFINDUNG

Erfindungsgemäß wird ein Verfahren zur Herstellung einer individuellen Sattelschale in Anspruch 1 wiedergegeben. Das Verfahren umfasst vorzugsweise folgende Schritte: Ermitteln einer individuellen Ausdehnung einer Prostata. Herstellen der individuellen Sattelschale mit einer Ausnehmung zur Aufnahme und Entlastung eines Genitalbereichs, der die Prostata umfasst, wobei die Abmessung und/oder Geometrie der Ausnehmung in Abhängigkeit der Ausdehnung des Genitalbereichs, insbesondere der Prostata, ermittelt werden kann.

Der Begriff Ausdehnung soll dabei vorzugsweise die Bedeutung des Maßes einer räumlichen Dimension eines Objekts umfassen. Insbesondere soll Ausdehnung das Volumen, die räumliche Form, Dichte, Gewicht oder die Abmessung, wie Höhe, Länge und Breite umfassen. Der Genitalbereich umfasst insbesondere Penis, Hodensack, Hoden, Nebenhoden, Samenleiter, Prostata vorgenannte Organe umgebendes Gewebe und Gefäße.

Das erfindungsgemäße Herstellverfahren ermöglicht insbesondere Männern mit Prostatavergrößerung mit geringen Beschwerden bzw. Risiken Fahrrad zu fahren. Die so hergestellte Sattelschale kann ein Taubheitsgefühl und Minderdurchblutung im Penis verringern. Durch die individuelle Ausnehmung kann eine große Sattelauflagefläche erreicht werden, die insbesondere im Unterschied zu bekannten Sätteln mit großem, länglichen Kanal, das Gewicht eines auf der Sattelschale sitzenden Fahrers gleichmäßiger und/oder großflächiger verteilt und dadurch eine schmerzhafte oder sogar eine krankheitsverursachende Druckbelastung, etwa auf Schambeinschaufeln des Fahrers, reduziert. Die so hergestellte Sattelschale vermag Periostitis bzw. Tendinopathien des Musculus piriformis zu verhindern bzw. reduzieren. Vor allem kann die so hergestellte Sattelschale das Gewicht des auf der Sattelschale sitzenden Fahrers auf dessen Sitzbeinhöcker verlagern. Die Prostataregion des auf der Sattelschale sitzenden Fahrers kommt vorzugsweise über der individuellen Ausnehmung zu liegen, wodurch ein Bereich an bzw. um der Prostata - auch englisch prostate pressure point bezeichnet - entlastet werden kann. Kurz gesagt kann durch das erfindungsgemäße Verfahren eine Sattelschale hergestellt werden, die eine verbesserte Druckverteilung für einen darauf sitzenden Fahrer bei gleichzeitiger Entlastung der individuellen Prostata des Fahrers erreichen kann. Die Individualisierung ist insbesondere deshalb von Vorteil, weil die Prostatagröße bzw. die Ausdehnung bestimmter Genitalbereiche individuell unterschiedlich sein können. Ist die Prostataausdehnung, -abmessung, bzw. -volumen ermittelt worden, so kann die Ausnehmung darauf angepasst werden.

Das Ermitteln der Ausdehnung erfolgt dabei vorzugsweise mittels eines bildgebenden Verfahrens, das ein Ultraschallmessverfahren und insbesondere eine transrektale Ultraschallmessung umfasst. Diese transrektale Ultraschallmessung kann dabei über den Enddarm erfolgen, wobei vorzugsweise in einem Abstand von in etwa einem Zentimeter über einem Schließmuskel gemessen wird. Der Verfahrensschritt der Ermittlung der Ausdehnung dient nicht der Diagnose zu einem etwaigen Heilzweck, sondern der Ermittlung von Daten bzw. physikalischer Größen, wie der Ausdehnung, des Volumens, der räumlichen Form, der Dichte, des Gewichts oder der Abmessung, wie Höhe, Länge und Breite um die Abmessung und/oder Geometrie der Ausnehmung in Abhängigkeit der Ausdehnung des Genitalbereichs, insbesondere der Prostata, ermitteln zu können.

Die Form der Prostata kann von ihrem Volumen abhängen und bei einem Volumen unter 80 ml einem Ellipsoid bzw. einer Kastanie ähneln, so ist in einer Sagittalebene die Länge der Prostata und einer Transversalebene die Breite, sowie die Höhe der Prostata bestimmbar. Daraus resultierend kann ihr Volumen näherungsweise mit folgender Formel bestimmt werden.

$$Volumen = (Länge \cdot Breite \cdot Höhe) \cdot \frac{\pi}{6}$$

Aus technischen Gründen kann die Länge der Prostata nur ungenau ermittelbar sein. Näherungsweise kann das Prostatavolumen aus der Breite und der Höhe in einer Transversalebene ermittelt werden.

$$Volumen = (Breite^2 \cdot Höhe) \cdot \frac{\pi}{6}$$

Ab einem Prostatavolumen von mehr als 80 ml kann ihre Form einer Kugel bzw. einer Kastanie ähneln. Daher wird insbesondere nur die Breite in der Transversalebene zur Ermittlung des Volumens herangezogen. Damit ergibt sich für die Bestimmung des Prostatavolumens vorzugsweise:

$$Volumen = (Breite^3) \cdot \frac{\pi}{6}$$

Als Abmessungen für die Ausnehmung zur Aufnahme und Entlastung von unterschiedlichen Prostatavolumina sind Werte der folgenden Wertetabelle bevorzugt:

| Größenbereich | Volumen min. in ml | Volumen mittel in ml | Volumen max. in ml | Erste Abmessung der Ausnehmung in mm | Zweite Abmessung der Ausnehmung in mm |
|---|---|---|---|---|---|
| 1 | 0 | 10 | 20 | 20 | 25 |
| 2 | 20 | 30 | 40 | 25 | 30 |
| 3 | 40 | 50 | 60 | 30 | 35 |
| 4 | 60 | 75 | 90 | 35 | 40 |
| 5 | 90 | 105 | 120 | 40 | 45 |

Die erste Abmessung ist vorzugsweise eine Länge der Ausnehmung und die zweite Abmessung eine Breite der Ausnehmung. Diese Zuordnung kann jedoch auch umgekehrt von Vorteil sein. Die Herstellkosten, z.B. Werkzeugkosten, können verringert werden, wenn die verschiedenen Prostatavolumina in Gruppen zusammengefasst werden und für diese Gruppen fest Abmessungen der Ausnehmung hergestellt werden. Insbesondere eine Einteilung in fünf Größenbereich ist bevorzugt.

Die Zuordnung der Prostatavolumina Volumen min., Volumen mittel und Volumen max. in die fünf Größenbereiche sowie zu den Abmessungen ist nicht abschließend, sondern kann auch in feinere Größenbereiche unterteilt bzw. nach unten oder oben erweitert sein. So ist auch eine Ausnehmung für ein Prostatavolumen größer 120 ml möglich, deren Abmessung bzw. Abmessungen kann durch Hochrechnung bzw. Interpolation aus der vorgenannten Werttabelle ermittelt werden.

Die vorgenannte Wertetabelle berücksichtigt vorteilhaft eine Sitzposition des auf der Sattelschale sitzenden Fahrers, sowie eine Druckverteilung unter Berücksichtigung der Sitzposition eines nach vorne zu einem Lenker geneigten Fahrradfahrers. Dies kann zu einer leichten Schrägstellung des Beckens nach vorne führen, wodurch vermehrt Druck auf einen Prostata-Druckpunkt (prostate pressure point) ausgeübt wird, der mittels der Sattelschale vorteilhaft aufgenommen und entlastet wird.

In einer bevorzugten Ausführungsform der Sattelschale ist diese mittels eines Spritzgussverfahrens unter Verwendung eines Polymers, wie beispielsweise Polypropylen oder Acrylnitril-Butadien-Styrol-Copolymer (kurz: ABS) herstellbar. Die Einteilung der Prostatavolumina und damit der Ausnehmungsabmessungen in eine endliche Anzahl von Größenbereichen ermöglicht eine kostengünstige Herstellung von Spritzgusswerkzeugen.

Ferner wird erfindungsgemäß eine Sattelschale nach Anspruch 7 bereitgestellt. Die erfindungsgemäße Sattelschale hat vorzugsweise eine Ausnehmung zur Aufnahme und Entlastung eines Genitalbereichs, insbesondere der Prostata, wobei die Abmessung und/oder die Geometrie der Ausnehmung nach dem erfindungsgemäßen Verfahren gebildet ist. Die Geometrie umfasst insbesondere einen Kreis, eine Ellipse, eine Kastanienform und ein gleichschenkliges Dreieck.

Noch bevorzugter ist die Ausnehmung in Form eines so genannten gleichschenkliges Bogen-Dreieck ausgebildet. Das gleichschenkelige Bogen-Dreieck hat konvexe d.h. nach außen gewölbte Seiten und kann außerdem abgerundete Ecken aufweisen. Um ein angenehmes Sitzgefühl und ein verbessertes Spritzgießen zu ermöglichen, kann die Ausnehmung angefaste oder abgerundete Kanten aufweisen. Ein umlaufender Rand der Ausnehmung kann konvex d.h. nach außen gewölbt sein. Die Ausnehmung berücksichtigt insbesondere die anatomische Ausbildung und Größe der Prostata und kann eine schädigende Druckresonanz und damit einhergehende Schmerzen bei einem darauf sitzenden Fahrer verringern.

In einer vorteilhaften Weiterbildung der Sattelschale umfasst die Abmessung und/oder Geometrie eine Länge in einer ersten Achse, die vorzugsweise von der Sitzebene zur Sattelnase bzw. in einer Sagittalebene verläuft, und eine Breite in einer zweiten Achse der Sattelschale, die vorzugsweise rechtwinkelig zur ersten Achse bzw. in einer Transversalebene verläuft.

Als weiterhin vorteilhaft gilt, dass ein Bereich der Sattelschale als Sitzfläche ausgebildet ist und eine zu einer Sitzebene und einer Sattelnase hin gewölbte Fläche aufweist. Die gewölbte Fläche umfasst dabei vorzugsweise ein Sattelpolster und ist insbesondere aus einem Kunststoffmaterial, etwa einem thermoplastischen Elastomer wie beispielsweise Polyurethan, gebildet. In einer weiteren Ausführungsform kann das Sattelpolster auch mit einem Bezugsstoff versehen sein.

In einer weiteren Ausführungsform der Erfindung ist entlang einer Längsachse der Sattelschale eine Vertiefung ausgebildet. Darin ist in einem vorbestimmten Abstand zu einer hinteren Sattelkante ein Zentrum der Ausnehmung vorzugsweise so angeordnet, dass es im Bereich eines Dammes eines darauf sitzenden Fahrers zu liegen kommt. Dieses Zentrum kann als sogenannter Fixpunkt für die Abmessungen und/oder Geometrien verstanden werden. Der vorbestimmte Abstand zwischen der hinteren Sattelkante und dem Fixpunkt der Ausnehmung beträgt in der bevorzugten Ausführungsform 80 bis 100 mm, insbesondere 90 mm.

Ferner wird ein erfindungsgemäßer Sattel gemäß Anspruch 14 vorgeschlagen, der insbesondere die erfindungsgemäße Sattelschale, ein Gestell und zwei Sattelstreben aufweist. Die erfindungsgemäße Sattelschale ist vorzugsweise auf das Gestell geklebt. Die Sattelschale kann zur Verbesserung des Sitzkomforts mit einer Moosgummischicht überzogen sein.

Außerdem wird ein erfindungsgemäßes Fahrrad mit dem erfindungsgemäßen Sattel vorgeschlagen. Grundsätzlich kann der erfindungsgemäße Sattel jede Art von Sitz, Stuhl oder Sitzvorrichtung für jede Art von Fahrzeug oder Möbel umfassen.

KURZBESCHREIBUNG DER ZEICHNUNGEN

Ausführungsbeispiele der Erfindung werden anhand der Zeichnungen und der nachfolgenden Beschreibung erläutert. Es zeigen:

Fig. 1     eine Schnittdarstellung eines männlichen Genitalbereichs;
Fig. 2     ein Verfahren gemäß einem Ausführungsbeispiel der Erfindung;
Fig. 3     eine Draufsicht auf eine Oberseite einer Sattelschale gemäß einem weiteren Ausführungsbeispiel der Erfindung;
Fig. 4     eine perspektivische Darstellung der Oberseite der Sattelschale gemäß einem weiteren Ausführungsbeispiel der Erfindung;
Fig. 5     eine Draufsicht auf eine hintere Sattelkante der Sattelschale gemäß der Erfindung;
Fig. 6A    ein Diagramm mit einem Zusammenhang zwischen einer ersten Abmessung der Ausnehmung 30 zum Prostatavolumen 44 gemäß einem weiteren Ausführungsbeispiel der Erfindung; und
Fig. 6B    ein Diagramm mit einem Zusammenhang zwischen einer zweiten Abmessung der Ausnehmung 30 zum Prostatavolumen 44 gemäß einem weiteren Ausführungsbeispiel der Erfindung.

## AUSFÜHRUNGSFORMEN DER ERFINDUNG

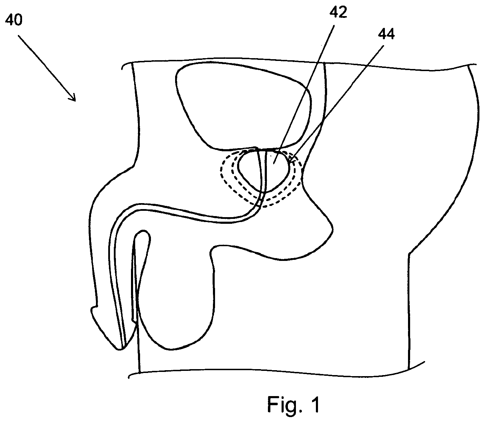

Figur 1 zeigt eine Schnittdarstellung eines männlichen Genitalbereichs 40 sowie eine Prostata 42, als ein pyramidenförmiges, ellipsoides bzw. Kastanienförmiges Organ, welches unterhalb einer Harnblase vor einem Enddarm lokalisiert ist. Ein Volumen 44 der Prostata 42 variiert je nach Alter und Zustand zwischen etwas weniger als 20 ml bis zu 120 ml.

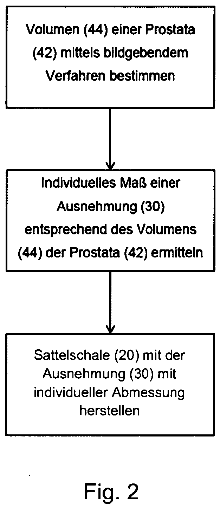

Figur 2 zeigt Schritte eines Verfahrens zur Herstellung einer individuellen Sattelschale 20 gemäß einem ersten Ausführungsbeispiel der Erfindung. Hierbei wird in einem ersten Schritt die Abmessung und daraus das Volumen 44 der Prostata 42 mittels einer transrektalen Ultraschallmessung ermittelt. Anschließend wird basierend auf dem Volumen 44 die Abmessung einer Ausnehmung 30 für die Aufnahme und Entlastung des der Prostata 42 ermittelt. Mit der so ermittelten individuellen Abmessung der Ausnehmung erfolgt die Herstellung der individuellen Sattelschale 20 mittels eines Spritzgussverfahrens.

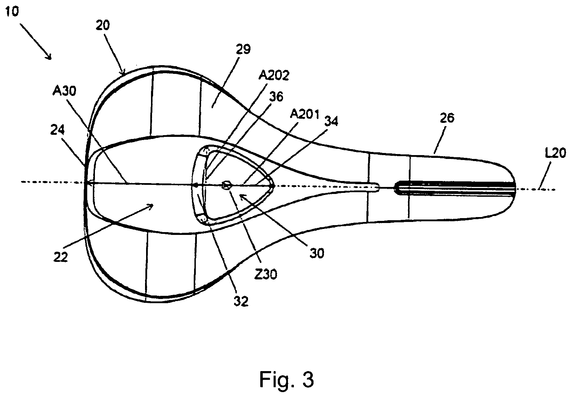

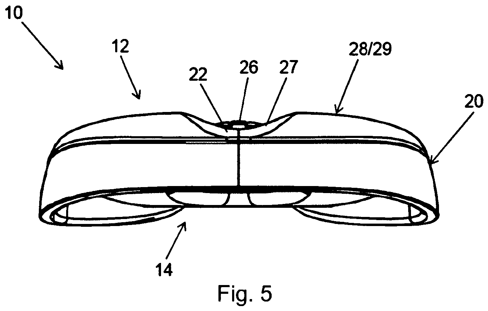

Figur 3 zeigt eine Draufsicht auf einen Sattels 10, der eine Sattelschale 20 umfasst. Der Sattel 10 weist eine Oberseite 12 und eine dieser gegenüberliegende Unterseite 14 (siehe Figur 5) auf. Ferner umfasst der Sattel 10 eine zur Aufnahme und Entlastung des Genitalbereichs 40 ausgebildeten Ausnehmung 30. Eine Länge 34 der Ausnehmung 30 verläuft dabei in Richtung einer ersten Achse A201 und eine Breite 36 in Richtung einer zweiten Achse A202 der Sattelschale 20. Die Ausnehmung 30 durchdringt die Sattelschale 20 und ist als gleichschenkliges Dreieck ausgebildet. Ein umlaufender Rand 32 der Ausnehmung 30 weist dabei eine konvexe d.h. nach außen gewölbte Form auf. Dadurch lässt sich die anatomische Ausbildung und Größe der Prostata 42 berücksichtigen und verhindert somit eine schädigende Druckresonanz und damit einhergehende Schmerzen im Genitalbereich 40 eines auf dem Sattel 10 sitzenden Fahrers.

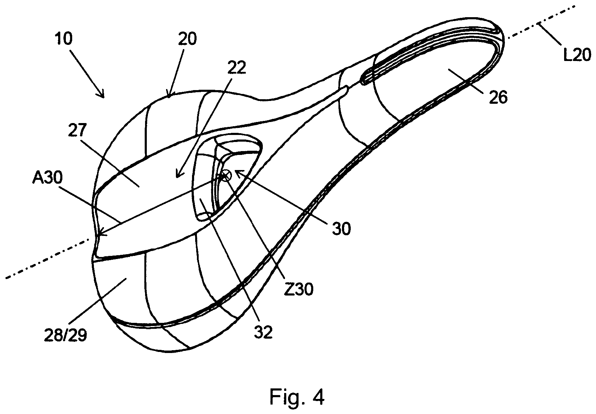

Weiter zeigt die Figur 3 einen als Sitzfläche ausgebildeten Bereich der Sattelschale 20 und eine zu einer Sitzebene 28 (siehe Figur 5) und einer Sattelnase 26 hin gewölbte Fläche 27 (siehe Figur 4). Die gewölbte Fläche 27 bildet dabei ein Sattelpolster 29. Das Sattelpolster 29 ist bevorzugt aus einem thermoplastischen Elastomer wie beispielsweise Polyurethan gebildet. Das Sattelpolster 29 kann zudem mit einem Bezugsstoff versehen sein. Das Sattelpolster 29 in seiner Form so gestaltet, dass die Prostata 42 im Dammbereich entlastet und der Druck - resultierend aus dem jeweiligen Körpergewicht des Fahrers - somit auf die Sitzbeinhöcker abgegeben wird.

Im Sattelpolster 29 selbst ist entlang einer Längsachse L20 eine Vertiefung 22 ausgebildet, welche sich zur Sattelnase 26 hin verjüngt. Ein Zentrum Z30 der Ausnehmung 30 ist in dieser Vertiefung 22 in einem vorbestimmten Abstand A30 zu einer hinteren Sattelkante 24 ausgebildet. Der Abstand A30 zwischen der hinteren Sattelkante 24 und dem Zentrum Z30 der Ausnehmung 30 beträgt 90 mm. Die aus der Lage des Zentrums Z30, sowie der Länge 34 und der Breite 36 der Ausnehmung 30 basierende Geometrie ist entsprechend der anatomischen Gegebenheiten der Prostata 42, beim Sitzen auf dem Sattel 10 als gleichschenkeliges Dreieck bzw. in Kastanienform gewählt.

Die perspektivische Darstellung der Oberseite 12 der Sattelschale 20 der Figur 4, zeigt die in der Figur 3 beschriebene Vertiefung 22, in der die Ausnehmung 30 ausgebildet ist, sowie die zur Sitzebene 28 und zur Sattelnase 26 hin gewölbt ausgebildete Fläche 27.

Ferner verdeutlicht die in der Figur 5 dargestellte Draufsicht der hinteren Sattelkante 24 der Sattelschale 20 die im Sattelpolster 29 ausgebildete Vertiefung 22, sowie die zu der Sitzebene 28 und der Sattelnase 26 hin verlaufende gewölbte Fläche 27.

Der Sattel 10 mit der Sattelschale 20 weist zudem wenigstens zwei Sattelstreben (in den Figuren nicht dargestellt) auf, die bevorzugt aus einer Stahllegierung mit hoher Zugfestigkeit, wie beispielsweise Chrom-Molybdän-Stahl, gebildet sind.

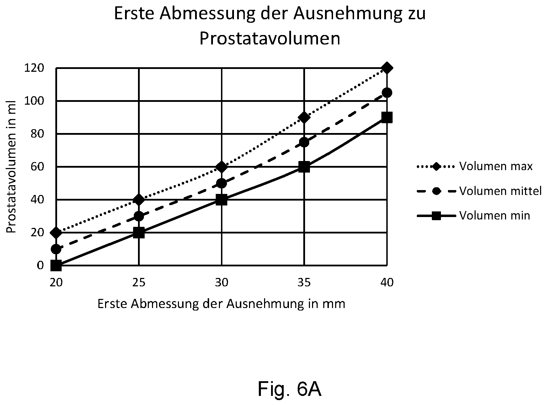

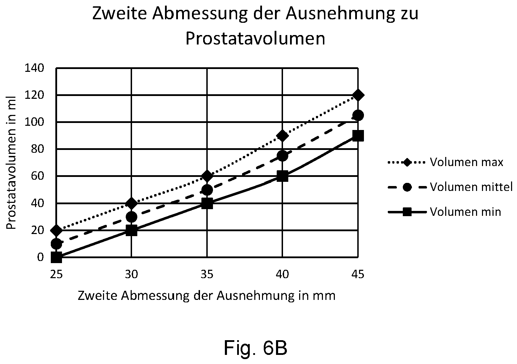

Figur 6A zeigt ein Diagramm, das einen Zusammenhang zwischen einer ersten Abmessung der Ausnehmung 30 zum Prostatavolumen 44 veranschaulicht. Figur 6B zeigt ein Diagramm, das einen Zusammenhang zwischen einer zweiten Abmessung der Ausnehmung 30 zum Prostatavolumen 44 veranschaulicht. Die Diagramme in Fig. 6A, 6B zeigen jeweils Kurven für Volumen- bzw. Größenbereiche, nämlich Volumen max., Volumen mittel und Volumen min. Die erste Abmessung entspricht der Länge 34 und die zweite Abmessung entspricht der Breite 36. Gemäß eines weiteren Ausführungsbeispiels entspricht die erste Abmessung der Breite 36 und die zweite Abmessung entspricht der Länge 34.

## BEZUGSZEICHENLISTE

10   Sattel
12   Oberseite
14   Unterseite

20   Sattelschale
22   Vertiefung

24 Sattelkante
26 Sattelnase
27 gewölbte Fläche
28 Sitzebene/Sitzfläche
29 Sattelpolster

30 Ausnehmung
32 Rand
34 Länge (von 30)
36 Breite (von 30)

40 Genitalbereich
42 Prostata
44 Volumen (von 42)

A30 Abstand (zwischen 24 und 30)
A201 (erste) Achse (von 20)
A202 (zweite) Achse (von 20)
L20 Längsachse (von 20)
Z30 Zentrum (von 30)

**Patentansprüche**

1. Verfahren zur Herstellung einer individuellen Sattelschale (20) mit folgenden Schritten:

   Ermitteln einer individuellen Ausdehnung einer Prostata (42) und Herstellen der individuellen Sattelschale (20) mit einer Ausnehmung (30) zur Aufnahme und Entlastung eines Genitalbereichs (40), der die Prostata (42) umfasst,
   wobei die Abmessung und/oder Geometrie der Ausnehmung (30) in Abhängigkeit der Prostata (42) ermittelt wird.

2. Verfahren nach Anspruch 1, **dadurch gekennzeichnet, dass** das Ermitteln der Ausdehnung mittels eines bildgebenden Verfahrens erfolgt.

3. Verfahren nach Anspruch 2, **dadurch gekennzeichnet, dass** das zur Ermittlung der Ausdehnung genutzte bildgebende Verfahren ein Ultraschallmessverfahren umfasst.

4. Verfahren nach einem der vorhergehenden Ansprüche, **dadurch gekennzeichnet, dass** die Abmessung der Ausnehmung (30) zwischen 20 und 45 mm liegt.

5. Verfahren nach einem der vorhergehenden Ansprüche, **dadurch gekennzeichnet, dass** die Abmessung der Ausnehmung (30) aus einem der fünf folgenden Größenbereiche ermittelt wird:

   ein erster Größenbereich für Prostatavolumina von kleiner 20 ml entspricht einer ersten Ausnehmungsabmessung von 20 mm und/oder einer zweiten Ausnehmungsabmessung von 25 mm,
   ein zweiter Größenbereich für Prostatavolumina von 20 ml bis 40 ml entspricht einer ersten Ausnehmungsabmessung von 25 mm und/oder einer zweiten Ausnehmungsabmessung von 30 mm,
   ein dritter Größenbereich für Prostatavolumina von 40 ml bis 60 ml entspricht einer ersten Ausnehmungsabmessung von 30 mm und/oder einer zweiten Ausnehmungsabmessung von 35 mm,
   ein vierter Größenbereich für Prostatavolumina von 60 ml bis 90 ml entspricht einer ersten Ausnehmungsabmessung von 40 mm und/oder einer zweiten Ausnehmungsabmessung von 45 mm und
   ein fünfter Größenbereich für Prostatavolumina von 90 ml bis 120 ml entspricht einer ersten Ausnehmungsabmessung von 40 mm und/oder einer zweiten Ausnehmungsabmessung von 45 mm.

6. Verfahren nach einem der vorhergehenden Ansprüche, **dadurch gekennzeichnet, dass** die Sattelschale (20) mittels eines Spritzgussverfahrens hergestellt wird.

# EP 3 921 221 B1

**Claims**

1. A method for producing a custom saddle shell (20), comprising the following steps:

   determining an individual extent of a prostate (42) and producing the custom saddle shell (20) having a recess (30) for accommodating and relieving a genital region (40) that comprises the prostate (42),
   wherein the dimension and/or geometry of the recess (30) is determined on the basis of the prostate (42).

2. The method according to claim 1, **characterized in that** the extent is determined by means of an imaging method.

3. The method according to claim 2, **characterized in that** the imaging method used to determine the extent comprises an ultrasound measuring method.

4. The method according to any one of the preceding claims,
   **characterized in that** the dimension of the recess (30) is between 20 and 45 mm.

5. The method according to any one of the preceding claims,
   **characterized in that** the dimension of the recess (30) is determined from one of the following five size ranges:

   a first size range for prostate volumes of less than 20 ml corresponds to a first recess dimension of 20 mm and/or a second recess dimension of 25 mm,
   a second size range for prostate volumes from 20 ml to 40 ml corresponds to a first recess dimension of 25 mm and/or a second recess dimension of 30 mm,
   a third size range for prostate volumes from 40 ml to 60 ml corresponds to a first recess dimension of 30 mm and/or a second recess dimension of 35 mm,
   a fourth size range for prostate volumes from 60 ml to 90 ml corresponds to a first recess dimension of 40 mm and/or a second recess dimension of 45 mm, and
   a fifth size range for prostate volumes from 90 ml to 120 ml corresponds to a first recess dimension of 40 mm and/or a second recess dimension of 45 mm.

6. The method according to any one of the preceding claims,
   **characterized in that** the saddle shell (20) is produced by means of an injection molding process.

**Revendications**

1. Procédé pour la fabrication d'une coque de selle (20) individuelle comportant les étapes suivantes :

   détermination d'un gonflement individuel d'une prostate (42) et fabrication de la coque de selle (20) individuelle avec un évidement (30) permettant de recevoir et de soulager une zone génitale (40) qui comprend la prostate (42),
   dans lequel la dimension et/ou la géométrie de l'évidement (30) sont déterminées en fonction de la prostate (42).

2. Procédé selon la revendication 1, **caractérisé en ce que** la détermination du gonflement est effectuée au moyen d'un procédé d'imagerie.

3. Procédé selon la revendication 2, **caractérisé en ce que** le procédé d'imagerie utilisé pour déterminer le gonflement comprend un procédé de mesure par ultrasons.

4. Procédé selon l'une des revendications précédentes,
   **caractérisé en ce que** la dimension de l'évidement (30) est comprise entre 20 et 45 mm.

5. Procédé selon l'une des revendications précédentes,
   **caractérisé en ce que** la dimension de l'évidement (30) est déterminée à partir de l'une des cinq plages de tailles suivantes :

   une première plage de tailles pour des volumes de la prostate inférieurs à 20 ml correspond à une première dimension d'évidement de 20 mm et/ou à une seconde dimension d'évidement de 25 mm,

7

une deuxième plage de tailles pour des volumes de la prostate allant de 20 ml à 40 ml correspond à une première dimension d'évidement de 25 mm et/ou à une seconde dimension d'évidement de 30 mm,
une troisième plage de tailles pour des volumes de la prostate allant de 40 ml à 60 ml correspond à une première dimension d'évidement de 30 mm et/ou à une seconde dimension d'évidement de 35 mm,
une quatrième plage de tailles pour des volumes de la prostate allant de 60 ml à 90 ml correspond à une première dimension d'évidement de 40 mm et/ou à une seconde dimension d'évidement de 45 mm et
une cinquième plage de tailles pour des volumes de la prostate allant de 90 ml à 120 ml correspond à une première dimension d'évidement de 40 mm et/ou à une seconde dimension d'évidement de 45 mm.

6. Procédé selon l'une des revendications précédentes,
**caractérisé en ce que** la coque de selle (20) est fabriquée au moyen d'un procédé de moulage par injection.

Fig. 1

Volumen (44) einer Prostata
(42) mittels bildgebendem
Verfahren bestimmen

Individuelles Maß einer
Ausnehmung (30)
entsprechend des Volumens
(44) der Prostata (42) ermitteln

Sattelschale (20) mit der
Ausnehmung (30) mit
individueller Abmessung
herstellen

## Fig. 2

Fig. 3

Fig. 4

Fig. 5

Erste Abmessung der Ausnehmung zu Prostatavolumen

Fig. 6A

## Zweite Abmessung der Ausnehmung zu Prostatavolumen

Fig. 6B

**IN DER BESCHREIBUNG AUFGEFÜHRTE DOKUMENTE**

**In der Beschreibung aufgeführte Patentdokumente**

- EP 3022110 A1

- US 2006218809 A1